# EUROPÄISCHE PATENTSCHRIFT

(11) **EP 0 784 042 B1**
(45) Veröffentlichungstag und Bekanntmachung des Hinweises auf die Patenterteilung: **27.05.2009**
(21) Anmeldenummer: 97100109.4
(22) Anmeldetag: 07.01.1997
(51) Int. Cl.: C07B 37/00, C07C 41/48, C07C 67/293, C07C 403/10, C07D 311/72

(54) **Verwendung von Hydrogen-bis-(oxalato)borat**
Use of hydrogen bis(oxalato)borate
Usage d'hydrogenobis(oxalato)borate

(30) Priorität: 12.01.1996 CH 9696
(43) Veröffentlichungstag der Anmeldung: 16.07.1997
(73) Patentinhaber: DSM IP Assets B.V., 6411 TE Heerlen (NL)
(72) Erfinder: Fuerbringer, Claude, 4125 Riehen (CH)
(74) Vertreter: Steck, Melanie

(56) Entgegenhaltungen:
- EP-A- 0 314 309
- DE-A- 4 208 477
- US-A- 3 530 098
- CHEMISTRY LETTERS, Nr. 8, August 1982, TOKYO JP, Seiten 1185-1186, XP002028082 H. KOBAYASHI ET AL: "The first application of anion-catalyzed phase-transfer catalysis to Friedel-Crafts alkylation"
- JOURNAL OF ORGANOMETALLIC CHEMISTRY, Bd. 329, Nr. 1, 28.Juli 1987, LAUSANNE CH, Seiten 1-29, XP002028083 L. LAMANDÉ ET AL: "Structure et acidité de composés à atome de bore et de phosphore hypercoordonnés"

## Beschreibung

Die vorliegende Erfindung betrifft die Verwendung von Hydrogen-bis(oxalato)borat der Formel 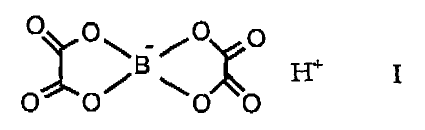 als Protonensäure-Katalysator in folgenden Kondensationsreaktionen. Friedel-Crafts-Kondensation von Benzoesäure-(4-hydroxy-2-methyl-naphthalen-1-yl)ester mit Isophytol zu Benzoesäure-[4-hydroxy-2-methyl-3-(3,7,11,15-tetramethyl-hexadec-2-enyl)-naphthalen-1-yl]ester (Monobenzoat des Dihydro-Vitamin K₁), Vinyletherkondensationen von Acetalen mit Vinyl- oder Propenylethern (zur Herstellung von Zwischenprodukten in der Synthese von Carotinoiden), und Acylierungen von Phenolen.

Als Beispiele von Vinyletherkondensationen von Acetalen mit Vinyl- oder Propenylethern zur Herstellung von Zwischenprodukten in der Synthese von Carotinoiden seien die Umsetzung von Acetaldehyd-dimethylacetal oder (E)-1,1,4,4-Tetramethoxy-but-2-en mit Methyl-propenylether oder von 13-(2,6,6-Trimethyl-cyclohexen-1-yl)-2,7,11-trimethyl-trideca-2,6,8,10,12-pentaen-4-in-1-al-diethylacetal mit Ethyl-vinylether, und als Beispiele von Acylierungen von Phenolen die Herstellung von d,l-α-Tocopherolacetat aus d,l-α-Tocopherol genannt.

Es wurden bereits verschiedene Verfahren zur Durchführung von Friedel-Crafts-Kondensationen in der Literatur beschrieben, welche unter Verwendung von Protonensäuren, wie Halogenwasserstoffsäuren, z.B. Chlorwasserstoffsäure, Trichloressigsäure und dgl., Lewis-Säuren, wie Aluminiumchlorid, Bortrifluorid, Eisenchlorid, Zinkchlorid und dgl., oder Gemischen von beiden Säurentypen, wie ein Gemisch von Zinkchlorid und einer starken Protonensäure und dgl., als Katalysatoren erfolgen.

Alle diese vorbekannten Verfahren weisen gewichtige Nachteile auf: so treten bei allen Verfahren Korrosionsprobleme auf, bei Verwendung von Bortrifluorid zusätzlich noch Toxizitätsprobleme mit den Addukten von Bortrifluorid sowie bei Verwendung von Eisen oder Zink eine heute nicht mehr akzeptable Belastung des Abwassers mit Eisen- oder Zinkionen.

Da Vinyletherkondensationen üblicherweise mit Lewis-Säuren durchgeführt werden, treten bei diesem Reaktionstyp ebenfalls Nachteile auf, wie sie oben für die Friedel-Crafts-Kondensationen mit Lewis-Säuren beschrie-ben sind.

DE 42 08 477 A1 beschreibt ein Verfahren zur Herstellung von dl-α-Tocopherol bzw. d1-α-Tocopherylacetat durch säurekatalysierte Umsetzung von 2,3,5-Trimethylhydrochinon mit Phytol oder Isophytol in einem Lösungsmittel bei erhöhter Temperatur und ggf. anschliessende Veresterung des erhaltenen Tocopherols mit Acetanhydrid, das dadurch gekennzeichnet ist, dass man die Umsetzung in Gegenwart von einer Mischung aus ortho-Borsäure und bestimmten aliphatischen Di- oder Tricarbonsäuren, vorzugsweise in Gegenwart von einer Mischung aus ortho-Borsäure und Oxalsäure durchführt.

US 3,530,098 beschreibt ein Verfahren zur Herstellung linearer faser- und folienbildender Polyester durch Polykondensation von Dicarbonsäurebisdiolestern bestimmter aromatischer Dicarbonsäuren, ggf. im Gemisch mit bis zu 20 Mol.% an bestimmten aliphatischen Dicarbonsäuren und/oder 1,2-Cyclobutandicarbonsäure, wobei die Diolkomponente der Dicarbonsäurebisdiolester ein Diol mit 2 bis 8 C-Atomen ist, in Gegenwart eines Katalysators, **dadurch gekennzeichnet, dass** als Polykondensationskatalysator eine katalytische Menge bestimmter gemischter Bor-haltiger Säureanhydride eingesetzt wird.

Der vorliegenden Erfindung liegt die Aufgabe zugrunde, für die oben genannten Kondensationsverfahren ein Katalysatorsystem zur Verfügung zu stellen, das die Nachteile der vorbekannten Arbeitsweisen nicht aufweist. Dazu ist es notwendig, dass das zur Verfügung gestellte Katalysatorsystem nicht korrosiv wirkt, nicht toxisch ist, die Umwelt nicht belastet, und die erwünschte Reaktion möglichst selektiv und in hohen Ausbeuten katalysiert. Darüber hinaus soll das Katalysatorsystem seine Wirkung bereits in wirklich nur katalytischen Mengen entfalten und sich leicht abtrennen lassen.

Im Rahmen der vorliegenden Erfindung wurde diese Aufgabe dadurch gelöst, dass man in den Kondensationsreaktionen Hydrogen-bis(oxalato)borat der obigen Formel I als Protonensäure-Katalysator verwendet.

Die Kondensationen können in Abhängigkeit vom Reaktionstyp (Friedel-Crafts-Kondensation, Vinyletherkondensation bzw. Acylierung von Phenolen) sowie den eingesetzten Ausgangsstoffen in Gegenwart oder Abwesenheit eines Lösungsmittels bei Temperaturen zwischen etwa 0°C und etwa 140°C durchgeführt werden. So wird die Friedel-Crafts-Kondensation zweckmässigerweise bei Temperaturen zwischen etwa 80°C und etwa 140°C, vorzugsweise zwischen etwa 85°C und etwa 120°C, besonders bevorzugt bei der Rückflusstemperatur des Reaktionsgemisches, durchgeführt. Die Vinyletherkondensationen werden hingegen zweckmässigerweise in Abwesenheit eines Lösungsmittels bei Temperaturen zwischen etwa 0°C und etwa 40°C, vorzugsweise zwischen etwa 15°C und etwa 25°C, besonders bevorzugt bei etwa Raumtemperatur, durchgeführt.

Als geeignete Lösungsmittel im Rahmen der vorliegenden Erfindung können aromatische Kohlenwasserstoffe, z.B. Toluol, Xylol und dergleichen, halogenierte aromatische Kohlenwasserstoffe, z.B. Chlorbenzol und dergleichen, aliphatische Carbonsäureester, z.B. Ethylacetat, Isopropylacetat und dergleichen, aliphatische Ether, z.B. Methyl-tert. butylether, Diisobutylether und dergleichen, sowie aliphatische und cyclische Ketone mit einem Siedepunkt zwischen etwa 80°C und etwa 140°C, z.B. Diethylketon, Methylisopropylketon, Cyclopentanon und dergleichen, genannt werden. Bevorzugt sind Toluol und Isopropylacetat.

Erfindungsgemäss kann die Kondensation in Gegenwart von etwa 0,1 bis etwa 6, bevorzugt etwa 0,2 bis etwa 4, besonders bevorzugt etwa 0,3 bis etwa 3 Mol% Hydrogen-bis(oxalato)borat als Katalysator durchgeführt werden (jeweils bezogen auf die massgebliche molare Menge des Ausgangsmaterials).

Das als Katalysator eingesetzte Hydrogen-bis(oxalato)borat der obigen Formel I ist aus Journal of Organometallic Chemistry 329, 1-29 (1987) bekannt. Es kann wie beispielsweise in Beispiel 1 beschrieben hergestellt werden, wobei es vorteilhafterweise nicht in situ hergestellt und eingesetzt, sondern in isolierter Form eingesetzt wird.

Die folgenden Beispiele erläutern die Erfindung, sollen jedoch keinerlei Einschränkung darstellen. Alle Temperaturen sind in Celsiusgraden angegeben.

### Beispiel 1

In einem 1l-Kolben wurden 13,96 g (150,36 mmol) Oxalsäure und 2,62 g (37,59 mmol) Dibortrioxid [oder 8,4 ml (75,2 mmol) Trimethylborat oder 3,7 ml (25,1 mmol) Trimethoxyboroxin] in 450 ml Toluol suspendiert und 6 Stunden unter Rückfluss gerührt, wobei das anfallende Reaktionswasser mittels Wasserabscheider kontinuierlich abgetrennt wurde. Das kalte Reaktionsgemisch wurde anschliessend filtriert, und der feste Rückstand am Vakuum getrocknet, wobei Hydrogen-bis(oxalato)borat in 70%iger Ausbeute erhalten wurde.

### Beispiel 2

In einem 500 ml-Sulfierkolben wurden 28,398 g (100 mmol) Benzoesäure-(4-hydroxy-2-methyl-naphthalen-1-yl)ester mit 127,4 mg (0,569 mmol) Hydrogen-bis(oxalato)borat in 170 ml Toluol vorgelegt. Das Reaktionsgemisch wurde auf 98° erwärmt und anschliessend innerhalb von 15 Sekunden mit 17,76 g (56,9 mmol) Isophytol versetzt .

Nach beendeter Zugabe wurde das Reaktionsgemisch noch 30 Minuten bei 98° gerührt. Die Ausbeute an Benzoesäure-[4-hydroxy-2-methyl-3-[(E)-3,7,11,15-tetramethyl-hexadec-2-enyl]-naphthalen-1-yl]ester beträgt 80% (berechnet auf Isophytol, direkte LC-Bestimmung aus dem Reaktionsgemisch anhand einer Eichkurve).

### Beispiel 3

In einem 200 ml-Sulfierkolben wurden unter Rühren 35,240 g (200 mmol) (E)-1,1,4,4-Tetramethoxy-but-2-en und 1,343 g (6 mmol) Hydrogen-bis (oxalato)borat vorgelegt. Innerhalb von 4 Stunden gab man anschliessend tropfenweise bei Raumtemperatur 28,840 g (400 mmol) Methyl-propenylether zu und liess nach beendeter Zugabe noch 30 Minuten nachreagieren.

Die Reaktionslösung wurde danach mit 300 ml Wasser und 2 ml 15%-iger Salzsäure versetzt, und das entstandene Methanol abdestilliert. Innerhalb von 15 Minuten gab man 12 ml 15%-ige Natronlauge zu und liess noch 30 Minuten bei 80° nachrühren. Das erkaltete Reaktionsgemisch wurde dann filtriert und getrocknet, wobei 2,7-Dimethyl-2,4,6-(E,E,E)-octatrien-dial in 67%-iger Ausbeute (LC-Analyse) erhalten wurde.

### Beispiel 4

In einem 100 ml-Sulfierkolben wurden 27,04 g (300 mmol) Acetaldehyd-dimethylacetal und 282 mg (1,26 mmol) Hydrogen-bis(oxalato)borat vorgelegt und dann 7,21 g (100 mmol) Methyl-propenylether innerhalb von 2 Stunden bei Raumtemperatur zugetropft. Nachdem man das Reaktionsgemisch eine Stunde auf 40° erwärmt hatte, liess man auf Raumtemperatur abkühlen, wobei man 1,1,3-Trimethoxy-2-methyl-butan erhielt; der Gehalt an 1,1,3-Trimethoxy-2-methyl-butan betrug nach GC-Analyse 70%.

### Beispiel 5

In einem 100 ml-Sulfierkolben wurde ein Gemisch aus 5,0 g (14,3 mmol) 13-(2,6,6-Trimethyl-cyclohexen-1-yl)-2,7,11-trimethyl-trideca-2,6,8,10,12-pentaen-4-in-1-al, 3,1 ml (18,2 mmol) Orthoameisensäure-triethylester, 0,8 ml Ameisensäureethylester und 89,5 mg (0,4 mmol) Hydrogen-bis (oxalato)borat vorgelegt. Das Reaktionsgemisch wurde 1 Stunde bei 15° gerührt, anschliessend bei dieser Temperatur tropfenweise mit 1,9 ml (19,7 mmol) Ethyl-vinylether versetzt und nochmals 15 Minuten nachgerührt. Die Ausbeute an 13,15,15-Triethoxy-3,7,12-trimethyl-1-(2,6,6-trimethyl-cyclohex-1-enyl)-pentadeca-1,3,5,7,11-pentaen-9-in betrug 80%.

### Beispiel 6

In einem 100 ml-Sulfierkolben wurde ein Gemisch von 43,00 g (100 mmol) Tocopherol, 11,40 g (110 mmol) Essigsäureanhydrid und 94 mg (0,5 mmol) Hydrogen-bis(oxalato)borat vorgelegt, und das Reaktionsgemisch eine Stunde unter ArgonAtmosphäre zum Rückfluss erhitzt. Nach dem Einengen am Rotationsverdampfer blieben 47,2 g rohes d,1-α-Tocopherolacetat zurück, das einen Gehalt von 87% aufwies; Ausbeute 92%.

## Patentansprüche

1. Verwendung von Hydrogen-bis(oxalato)borat der Formel 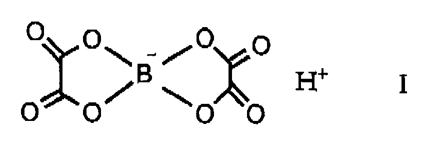 als Protonensäure-Katalysator in Kondensationsreaktionen, **dadurch gekennzeichnet, dass** es sich bei der Kondensation um eine Friedel-Crafts-Kondensation von Benzoesäure-(4-hydroxy-2-methyl-naphthalen-1-yl)ester mit Isophytol, um eine Vinyletherkondensation eines Acetals mit einem Vinyl- oder Propenylether oder um eine Acylierung eines Phenols handelt.

2. Verwendung gemäss Anspruch 1, **dadurch gekennzeichnet, dass** das Hydrogen-bis(oxalato)borat der Formel I in isolierter Form eingesetzt wird.

3. Verwendung gemäss Anspruch 1 oder 2, **dadurch gekennzeichnet, dass** 0,1 bis 6, bevorzugt 0,2 bis 4, besonders bevorzugt 0,3 bis 3, Mol% Hydrogen-bis(oxalato)borat als Katalysator eingesetzt wird.

4. Verwendung gemäss einem der Ansprüche 1 bis 3, **dadurch gekennzeichnet, dass** die Friedel-Crafts-Kondensation bei Temperaturen zwischen 80°C und 140°C, vorzugsweise zwischen 85°C und 120°C, besonders bevorzugt bei der Rückflusstemperatur des Reaktionsgemisches, erfolgt.

5. Verwendung gemäss einem der Ansprüche 1 bis 4, **dadurch gekennzeichnet, dass** als Lösungsmittel ein aromatischer Kohlenwasserstoff, ein halogenierter aromatischer Kohlenwasserstoff, ein aliphatischer Carbonsäureester, ein aliphatischer Ether oder ein aliphatisches oder cyclisches Keton mit einem Siedepunkt zwischen 85°C und 140°C eingesetzt wird.

6. Verwendung gemäss Anspruch 5, **dadurch gekennzeichnet, dass** als Lösungsmittel Toluol, Xylol, Chlorbenzol, Ethylacetat, Isopropylacetat, Methyl-tert.butylether, Diisobutylether, Diethylketon, Methylisopropylketon oder Cyclopentanon, vorzugsweise Toluol oder Isopropylacetat, eingesetzt wird.

7. Verwendung gemäss einem der Ansprüche 1 bis 3, **dadurch gekennzeichnet, dass** die Vinyletherkondensation bei Temperaturen zwischen etwa 0°C und etwa 40°C, vorzugsweise zwischen etwa 15°C und etwa 25°C, besonders bevorzugt bei etwa Raumtemperatur, erfolgt.

8. Verwendung gemäss Anspruch 7, **dadurch gekennzeichnet, dass** die Kondensation in Abwesenheit eines Lösungsmittels erfolgt.

9. Verwendung gemäss einem der Ansprüche 1 bis 3, 7 und 8, **dadurch gekennzeichnet, dass** Acetaldehyd-dimethylacetal mit Methylpropenylether umgesetzt wird.

10. Verwendung gemäss einem der Ansprüche 1 bis 3, 7 und 8, **dadurch gekennzeichnet, dass** (E)-1,1,4,4-Tetramethoxy-but-2-en mit Methylpropenylether umgesetzt wird.

11. Verwendung gemäss einem der Ansprüche 1 bis 3, 7 und 8, **dadurch gekennzeichnet, dass** 13-(2,6,6-Trimethylcyclohexen-1-yl)-2,7,11-trimethyl-trideca-2,6,8,10,12-pentaen-4-in-1-al-diethylacetal mit Ethylvinylether umgesetzt wird.

## Claims

1. Use of hydrogen bis(oxalato)borate of formula 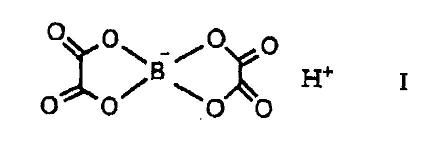 as protic acid catalyst in condensation reactions, **characterized in that** the condensation is a Friedel-Crafts condensation of 4-hydroxy-2-methylnaphthalen-1-yl benzoate with isophytol, a vinyl ether condensation of an acetal with a vinyl or propenyl ether, or an acylation of a phenol.

2. Use according to Claim 1, **characterized in that** the hydrogen bis(oxalato)borate of formula I is used in isolated form.

3. Use according to Claim 1 or 2, **characterized in that** 0.1 to 6, preferably 0.2 to 4 and more preferably 0.3 to 3 mol% of hydrogen bis(oxalato)borate is used as catalyst.

4. Use according to any one of Claims 1 to 3,
**characterized in that** the Friedel-Crafts condensation is carried out at temperatures between 80°C and 140°C and preferably between 85°C and 120°C and more preferably at the reflux temperature of the reaction mixture.

5. Use according to any one of Claims 1 to 4, **characterized in that** an aromatic hydrocarbon, a halogenated aromatic hydrocarbon, an aliphatic carboxylic ester, an aliphatic ether or an aliphatic or cyclic ketone having a boiling point between 85°C and 140°C is used as solvent.

6. Use according to Claim 5, **characterized in that** toluene, xylene, chlorobenzene, ethyl acetate, isopropyl acetate, methyl tert-butyl ether, diisobutyl ether, diethyl ketone, methyl isopropyl ketone or cyclopentanone, preferably toluene or isopropyl acetate, is used as solvent.

7. Use according to any one of Claims 1 to 3, **characterized in that** the vinyl ether condensation is carried out at temperatures between about 0°C and about 40°C, preferably between about 15°C and about 25°C and more preferably at about room temperature.

8. Use according to Claim 7, **characterized in that** the condensation is carried out in the absence of a solvent.

9. Use according to any one of Claims 1 to 3, 7 and 8, **characterized in that** acetaldehyde dimethyl acetal is reacted with methyl propenyl ether.

10. Use according to any one of Claims 1 to 3, 7 and 8, **characterized in that** (E)-1,1,4,4-tetramethoxybut-2-ene is reacted with methyl propenyl ether.

11. Use according to any one of Claims 1 to 3, 7 and 8, **characterized in that** 13-(2,6,6-trimethylcyclohexen-1-yl)-2,7,11-trimethyltrideca-2,6,8,10,12-pentaen-4-yn-1-al diethyl acetal is reacted with ethyl vinyl ether.

## Revendications

1. Utilisation de bis(oxalato)borate d'hydrogène de formule 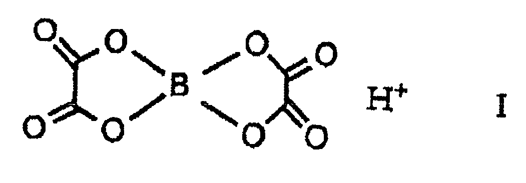 en tant que catalyseur de type acide protonique dans des réactions de condensation, **caractérisée en ce que** la condensation consiste en une condensation de Friedel et Crafts de benzoate de 4-hydroxy-2-méthyl-naphtalén-1-yle avec l'isophytol, d'une condensation d'éther vinylique d'un acétal avec un éther vinylique ou propénylique ou d'une acylation d'un phénol.

2. Utilisation selon la revendication 1, **caractérisée en ce que** le bis(oxalato)borate d'hydrogène de formule I est utilisé sous forme isolée.

3. Utilisation selon la revendication 1 ou 2,
**caractérisée en ce qu'**on utilise comme catalyseur de 0,1 à 6, de préférence de 0,2 à 4, de façon particulièrement préférée, de 0,3 à 3 % en moles de bis(oxalato)borate d'hydrogène.

4. Utilisation selon l'une quelconque des revendications 1 à 3, **caractérisée en ce que** la condensation de Friedel et Crafts s'effectue à des températures comprises entre 80 °C et 140 °C, de préférence entre 85 °C et 120 °C, de façon particulièrement préférée à la température de reflux du mélange réactionnel.

5. Utilisation selon l'une quelconque des revendications 1 à 4, **caractérisée en ce qu'**on utilise comme solvant un hydrocarbure aromatique, un hydrocarbure aromatique halogéné, un ester d'acide carboxylique aliphatique, un éther aliphatique ou une cétone aliphatique ou cyclique ayant un point de d'ébullition compris entre 85 °C et 140 °C.

6. Utilisation selon la revendication 5, **caractérisée en ce qu'**on utilise comme solvant le toluène, le xylène, le chlorobenzène, l'acétate d'éthyle, l'acétate d'isopropyle, l'oxyde de méthyle et de tert-butyle, l'éther diisobutylique, la diéthylcétone, la méthylisopropylcétone ou la cyclopentanone, de préférence le toluène ou l'acétate d'isopropyle.

7. Utilisation selon l'une quelconque des revendications 1 à 3, **caractérisée en ce que** la condensation d'éther vinylique s'effectue à des températures comprises entre environ 0 °C et environ 40 °C, de préférence entre environ 15 °C et environ 25 °C, de façon particulièrement préférée aux environs de la température ambiante.

8. Utilisation selon la revendication 7, **caractérisée en ce que** la condensation s'effectue en absence d'un solvant.

9. Utilisation selon l'une quelconque des revendications 1 à 3, 7 et 8, **caractérisée en ce que** l'on fait réagir de l'acétaldéhyde-diméthylacétal avec de l'oxyde de méthyle et de propényle.

10. Utilisation selon l'une quelconque des revendications 1 à 3, 7 et 8, **caractérisée en ce que** l'on fait réagir du (E)-1,1,4,4-tétraméthoxybut-2-ène avec de l'oxyde de méthyle et de propényle.

11. Utilisation selon l'une quelconque des revendications 1 à 3, 7 et 8, **caractérisée en ce que** l'on fait réagir du 13-(2,6,6-triméthylcyclohexén-1-yl)-2,7,11-triméthyltridéca-2,6,8,10,12-pentaén-4-in-1-al-diéthylacétal avec de l'éthyl-vinyléther.
